# EUROPEAN PATENT SPECIFICATION

(11) **EP 0 889 020 B1**
(45) Date of publication and mention of the grant of the patent: **14.11.2001**
(21) Application number: 97121992.8
(22) Date of filing: 12.12.1997
(51) Int. Cl.: C07C 51/347, C07C 57/58, C07C 69/65, C07C 17/32, C07C 22/04, C07B 37/04

(54) **A process for the production of benzene derivatives**
Verfahren zur Herstellung von Benzolderivaten
Procédé de fabrication de dérivés du benzène

(30) Priority: 04.07.1997 KR 3104097
(43) Date of publication of application: 07.01.1999
(73) Proprietor: Kolon Industries, Inc., Kwacheon-city, Kyunggi-do 427-040 (KR)
(72) Inventor: Park, Sang Hoo, Yongin-city Kyunggi-do (KR); Kim, Maeng Sup, Kangdong-gu, Seoul (KR); Cho, Sung Min, Guseong-myun, Yongin-city, Kyunggi-do (KR); Kim, Ki Sug, Suzi-Yeup, Yongin-city, Kyunggi-do (KR); Kim, Jeong Soo, Seoul (KR); Cho, Eun Jung, Gunpo-city, Kyunggi-do (KR); Choi, Tae Gun, Hwasung-gun, Kyunggi-do (KR); Park, In Soo, Kitheung-yeup, Yongin-city, Kyunggi-do (KR)
(74) Representative: TER MEER STEINMEISTER & PARTNER GbR

(56) References cited:
- DATABASE WPI Section Ch, Week 8149 Derwent Publications Ltd., London, GB; Class B05, AN 81-90313D XP002079846 -& JP 56 138140 A (SANKYO CO LTD) , 28 October 1981
- PARKANYI C., L. S. C. YEH HUANG, S. G. CHU, A. T. JEFFRIES: "trans-4-STILBENECARBOXYLATOALKYLPENTAAMMI NECOBALT(III) PERCHLORATES: SYNTHESIS AND INTRAMOLECULAR ENERGY TRANSFER" COLLECT. CZECH. CHEM. COMMUN., vol. 61, 1996, pages 342-353, XP002079839
- G. L. STAHL, R. WALTER, C. W. SMITH: "PREPARATION AND CHARACTERIZATION OF BEADED POLY(N-ACRYLYLPYRROLIDINE): BIDIRECTIONAL SYNTHESIS OF CYS-, HIS-, GLN-, OR GLU-CONTAINING POLYPEPTIDES" JOURNAL OF THE AMERICAN CHEMICAL SOCIETY, vol. 101, no. 18, 1979, pages 5383-5394, XP002079840
- KUBICZEK G. ET AL.: "EXPERIMENTELLER BEITRAG ZUR "BROMMETHYLIERUNG" AROMATISCHER VERBINDUNGEN"" MONATSHEFTE FÜR CHEMIE, vol. 81, 1950, pages 917-920, XP002079841
- H. C. BROWN, K. L. NELSON: "AN INTERPRETATION OF META ORIENTATION IN THE ALKYLATION OF TOLUENE. THE RELATIVE REACTIVITY AND ISOMER DISTRIBUTION IN THE CHLOROMETHYLATION AND MERCURATION OF BENZENE AND TOLUENE" JOURNAL OF THE AMERICAN CHEMICAL SOCIETY, vol. 75, 1953, pages 6292-6299, XP002079842
- MAILLARD ET AL.: "ANTI-INFLAMMATOIRES D RIV S DE L'ACIDE PH NYLAC TIQUE ; D RIV S SUBSTITU S PAR UN H T ROCYCLE SUR LE NOYAU PH NYLE" CHIMIE TH RAPEUTIQUE, vol. 8, 1973, pages 487-494, XP002079843
- STEPHEN H. ET AL.: "THE INTRODUCTION OF THE CHLOROMETHYL GROUP INTO THE AROMATIC NUCLEUS" JOURNAL OF THE CHEMICAL SOCIETY, vol. 117, 1920, pages 510-527, XP002079844
- H. TOMIOKA, TAKETSUJI K.: "FORMATION OF HEPTAFULVENE IN REACTIONS OF [(METHOXYCARBONYL)METHYL]PHENYLCARBENE IN THE GAS PHASES" JOURNAL OF ORGANIC CHEMISTRY, vol. 58, 1993, pages 4196-4197, XP002079845
- DATABASE WPI Section Ch, Week 9737 Derwent Publications Ltd., London, GB; Class B05, AN 97-399470 XP002079847 & JP 09 176086 A (DAITO KK), 8 July 1997

## Description

### BACKGROUND OF THE INVENTION

### Field of Invention

The present invention relates to a process for the production of benzene derivatives of chemical formula ( I ), which are used as intermediates of agricultural chemical, fine chemical products or pharmaceuticals such as anti-inflammatory analgesics.

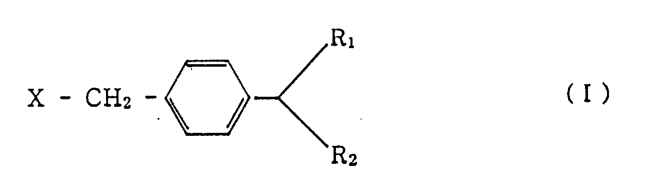

In chemical formula ( I ), X is halogen atom, R₁ is hydrogen atom or low alkyl radical of carbon number 1 - 6, R₂ is hydrogen atom or COOR₃ , and R₃ is hydrogen atom or low alkyl radical of carbon number 1 - 6.

Benzene derivatives of above chemical formula (I) are especially useful starting materials for the production of valuable 2-[4-(2-oxocyclo-pentylmethyl)phenyl] propionic acid, used as anti-inflammatory agent.

### Description of the Prior Art

Several methods have been reported to produce benzene derivatives of the aforesaid chemical formula (I).

According to JP 62-129250 and JP 62-155237, a method of reacting 2-(paramethyl)propionic acid and a halogenation agent in the presence of a radical generating agent has been disclosed.

Also in JP 56-138140, 2-(4-halomethylphenyl) propionic acid producing method of reacting 2-phenylpropionic acid and methylal in the presence of a strong Lewis acid such as aluminum chloride (AlCl₃), tin chloride (SnCl₄) has been disclosed.

Collect. Czech. Chem. Commun., vol. 61, 1996, pages 342-353 discloses a process for the synthesis of p-chloromethylphenylacetic acid, β-(p-chloromethylphenyl)propionic acid and γ-(p-chloromethylphenyl)butyric acid using as starting materials phenylacetic, β-phenylpropionic and γ-phenylbutyric acid, respectively. In this process formalin, hydrogen chloride and zinc chloride react with the starting material to yield the desired products. An alternative method for the synthesis of p-chloromethylphenylacetic acid and β-(p-chloromethylphenyl)propionic acid is disclosed as well, wherein the above cited starting materials react with chloromethyl methyl ether and stannic chloride.

Journal of the American Chemical Society, vol. 101, no. 18, 1979, pages 5383-5394, discloses a process for the synthesis of 4-(chloromethyl)phenylacetic acid wherein phenylacetic acid reacts with formaldehyde and HCl.

Monatshefte für Chemie, vol. 81, 1950, pages 917-920, discloses the synthesis of p-methylbenzylbromide from toluene, paraformaldehyde and sodium bromide.

Journal of the American Chemical Society, vol. 75, 1953, pages 6292-6299, discloses the reaction of toluene with a) formaldehyde, HCl and ZnCl₂ or b) with trioxymethylene, HCl and ZnCl₂ yielding p-methylbenzylchloride.

Chimie Thérapeutique, vol. 8, 1973, pages 487-494, discloses the reaction of ethyl 2-phenylpropionate with SnCl₄ and monochloro methyl ether yielding ethyl 2-(4-chloromethyl)phenylpropionate.

Journal of the Chemical Society, vol. 117, 1920, pages 510-527, discloses the reaction of toluene with ZnCl₂ and dichloromethyl ether to yield p-methylbenzylchloride and the reaction of toluene with dibromomethyl ether and ZnCl₂ to yield p-methylbenzylbromide.

JP 09 176 086 discloses the reaction of methyl phenylacetate with chloromethyl methyl ether and SnCl₄ to yield p-(chloromethyl)-phenylacetate.

As for the aforesaid conventional methods, a certain problem has been revealed that deterioration of purity in its final product due to plenty of by-product occurred during radical reaction. Consequently, some complicated refining process is required to improve the purity of final product. Besides in order to produce 2-(para-methyphenyl)propionic acid, as the starting material, several steps of chemical reaction should be passed through. And this fact means complicating the production process along with high product cost.

Therefore, it is required to revise a method of producing benzene derivatives of chemical formula ( I ) which has supreme purity from commercialized initiation substances.

### SUMMARY OF THE INVENTION

The present invention relates to a process for the production of benzene derivatives of chemical formula ( I ), which are used as intermediates of agricultural chemical, fine chemical products or pharmaceuticals such as anti-inflammatory analgesics. More particularly, the present invention relates to a process for the production of benzene derivatives expressed in below chemical formula ( I ) characterized by making reaction between a compound of chemical formula ( II) and a hydroformylating agent in the presence of a halogenation agent and in the presence of a phase transfer catalyst;

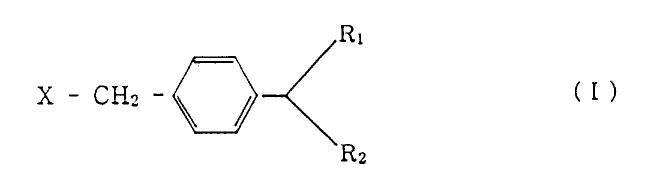

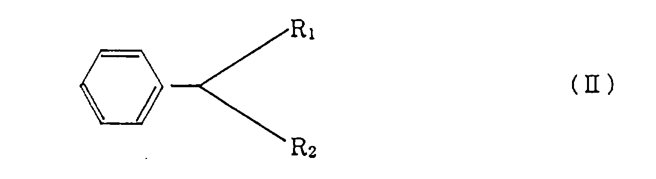

In chemical formulae (I) and (II), X is halogen atom, R₁ is hydrogen atom or low alkyl radical of carbon number 1 - 6, and R₂ is hydrogen atom or COOR₃ , and R₃ is hydrogen atom or low alkyl radical of carbon number 1 - 6.

### DESCRIPTION OF THE PREFERRED EMBODIMENT

A detailed illustration of the present invention will be followed hereinafter.

The reaction of the present invention is performed by a certain reaction course expressed in below reaction formula 1.

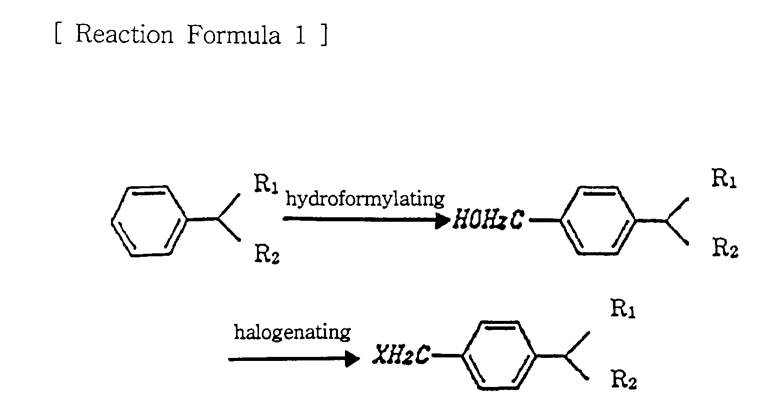

In above reaction formula, X is halogen atom, R₁ is hydrogen atom or low alkyl radical of carbon number 1 - 6, R₂ is hydrogen atom or COOR₃, and R₃ is hydrogen atom or low alkyl radical of carbon number 1 - 6.

As the above mentioned reaction could be performed in wide conditions, reaction time and purity of produced compounds will be varying depending on used quantity of hydroformylating agents and halogenation agents. Therefore, it is desirable that the above mentioned reaction is performed during 2 - 30 hours at 40 - 140 °C.
More desirably, the reaction will be performed during 10 - 15 hours at 80 - 120 °C.

In present invention, examples of hydroformylating agents are a polymer capable of generating formaldehyde in gaseous state or in solution state, paraformaldehyde, formaline, trioxane or methylal.
A desirable quantity of the said hydroformylating agent to be added is 1 - 10 equivalent per 1 equivalent of compound of chemical formula(II), the starting substance. More desirably, the quantity of hydroformylating agent to be added is 2 - 4 equivalent per 1 equivalent of compound of chemical formula(II), the starting substances. In case, the added quantity is less than 1 equivalent, reaction could not be made easily. And if the added quantity is more than 10 equivalent, economic effectiveness could be lowered.

On the other hand, examples of halogenation agents are hydrochloric acid, hydrobromic acid, hydroiodic acid, or metal halide in the presence of an acid. In the halogenation agents, examples of metal halide series are sodium chloride, sodium bromide, sodium iodide, potassium chliride, potassium bromide, potassium iodide, lithium chloride, or lithium bromide.
A desirable quantity of said halogenation agent to be added is 1.2 - 20 equivalent per 1 equivalent of compound of chemical formula(II), the starting material. More desirably, the quantity of halogenation agent to be added is 5 - 10 equivalent. In case, the added quantity is less than 1.2 equivalent, reaction could not be made easily. And if the added quantity is more than 20 equivalent, economic effectiveness could be lowered.

In present invention, this reaction is performed in the presence of a phase-transfer catalyst. Examples of phase transfer catalysts are tetraalkylammonium halides, such as tetramethylammonium bromide, tetramethylammonium chloride, methyl triethylammonium bromide, benzyl triethylammonium chloride, tetradecyl trimethylammonium bromide, hexadecyl trimethylammonium bromide, or phenyl trimethylammonium chloride. The quantity of phase-transfer catalysts added corresponds approximately to 2-20 mole % of phase-transfer catalysts, based on the quantity of starting materials employed.

In the mean time, the above mentioned reaction could be performed with or without a solvent system, which is inert under the reaction conditions. The solvent systems are composed of organic solvent, or mixtures of organic solvent and organic acid, or organic acid and water, or mixture of organic solvent and organic solvent and organic acid and water. Examples of organic solvents are methylene, chloroform, carbontertrachloride, 1,2-dichloroethane, acetonitrile, 4-dioxane, tetrahydrofurane, cyclohexane, N,N-dimethylformamide or N,N-dimethylacetamide. And examples of organic acids are sulfuric acid, acetic acid, phosphoric acid, trifluoroacetic acid, formic acid, methansulfonic acid or trifluoromethansulfonic acid.
A desirable quantity of said solvent is 0 - 50 equivalent per 1 equivalent of compound of chemical formula(II), the starting substance.

Followings are used for the compound of chemical formula (II) ; e.g. toluene, phenylacetic acid, 2-phenylpropionic acid, 2-phenylpropionic acidmethylester.

When the said reaction comes to an end, the generated compound will be crystallized by adding solvent or purified by using slilcagel column chromatography method after it has been extracted and concentrated.

In chemical formulae (I) and (II), low alkyl radical means an alkyl radical of linear chain or branched chain comprising methyl, ethyl, propyl, isopropyl, n-butyl, and isobutyl, and the halogen atom means chlorine, bromine, fluorine and iodine.

Examples of benzene derivatives of chemical formula ( I ) are 2-(4-chloromethylphenyl) propionic acid, 2-(4-chloromethylphenyl) propionic acid methyl ester, 2-(4-chloromethylphenyl) propionic ethyl ester, 2-(4-bromomethylphenyl) propionic acid, 2-(4-bromomethylphenyl) propionic methyl ester or, 2-(4-bromomethylphenyl) propionic ethyl ester.

Benzene derivatives of chemical formula (I) produced in the process of present invention are used as important intermediates of medicine, agricultural chemical, or fine chemical products.

Benzene derivatives of chemical formula (I) comprise optical isomers based on non-symmetrical carbon when R₁ and R₂ are different from each other, and all optical isomers and their mixtures are expressed in a singular structural formula for conveniency.

The present invention will be discussed more detail by examples stated below. But, the present invention is not limited to examples.

### EXAMPLE 1

Toluene(16g) was added to a mixture of acetic acid(25ml) and hydrobromic acid(95ml), followed by paraformaldehyde(12g) and benzyltriethylammonium bromide (1.0g). The mixture was then heated to 70°C and stirred well at the same temperature for 24 hours. After cooling to room temperature, the mixture was extracted with dichloromethane and then concentrated to give oil residue. The oil residue was distilled under vacuum to give the target product, bromomethyltoluene (29g).
Result of measurement by NMR(CDCl₃ , ppm) was 4.5(2H, s), 7.3(5H, m).

### EXAMPLE 2

Toluene(18g) was added to a mixture of acetic acid(25ml) and hydrobromic acid(95ml), followed by trioxane (6.6g) and benzyltriethylammonium bromide (2.0g). The mixture was then heated to 70°C and stirred well at the same temperature for 20 hours. After cooling to room temperature, the mixture was extracted with dichloromethane and then concentrated to give oil residue. The oil residue was distilled under vacuum to give the target product, 4-bromomethyltoluene (30.0g).
Result of measurement by NMR(CDCl₃ , ppm) was 2.4(3H, s), 4.5(2H, s), 7.3(4H, dd).

### EXAMPLE 3

Phenylacetic acid (27g) was added to a mixture of acetic acid(25ml) and hydrochloric acid(95ml), followed by paraformaldehyde(12g) and hexadecyltrimethylammonium bromides (3.0g). The mixture was then heated to 70°C and stirred well at the same temperature for 26 hours.

After cooling to room temperature, the mixture was extracted with dichloromethane and then concentrated to give oil residue. The oil residue was distilled under vacuum to give the target product, 4-(chloromethyl) phenylacetic methylester (12.0g).
Result of measurement by NMR(CDCl₃, ppm) was 3.5(2H, s), 3.6(3H, s) 4.5(2H, s), 7.3(4H, dd).

### EXAMPLE 4

2-phenylpropionic acid (7.5) was added to a mixture of formic acid(7ml) and hydrochloric acid(20ml), followed by trioxane (4.5g) and benzyltriethylammonium bromide(0.5g). The mixture was then heated to 120°C and stirred well at the same temperature for 30 hours. After cooling to room temperature, the mixture was extracted with ethylacetic acid and then concentrated to give oil residue. The oil residue was distilled and refined under vacuum to give the target product, 2-[(4-chloromethyl)phenyl] propionic acid (2.3g).
Result of measurement by NMR(CDCl₃ , ppm) was 1.5(3H, d), 3.7(1H, q), 4.5(2H, s), 7.3(4H, dd).

### EXAMPLE 5

Pour 12.0g 2-{(4-chloromethyl)phenyl}propionic and 1.5.0g sodium iodide into 130ml of methylethylketone, then be refluxed for 10 hours. Eliminate solvent under depressuring, and extract by adopting ether. After wash the ether layer with sodiumthiosulfate solution and water, 13.4g of 2- {(4-iodomethyl)phenyl} propionic acid was obtained by dry up and concentration.
Result of measurement by NMR(CDCl₃, ppm) was 1.5(3H, d), 3.7(1H, q), 4.5(2H, s), 7.3(4H, dd).

### EXAMPLE 6

2-phenylpropionic acid - (8.2g) was added to a mixture of acetic acid(7ml) and hydrobromic acid(40ml), followed by trioxane (4.5g) and hexadecyltrimethylammonium bromide (0.5g). The mixture was then heated to 120°C and stirred well at the same temperature for 19 hours. After cooling to room temperature, the mixture was extracted with ethylacetic acid and then concentrated to give oil residue. The oil residue was distiilled and refined under vacuum to give the target product, 2-[(4-bromomethyl) phenyl] propionic acid (1.5g).
Result of measurement by NMR(CDCl₃, ppm) was 1.5(3H, d), 3.7(1H, q), 4.5(2H, s), 7.3(4H, dd).

### EXAMPLE 7

162g of hydrochloric acid, 75g of potassium chloride, 3g of tetramethyl ammonium chloride, 18g of paraformaldehyde were poured orderly into 2-phenylpropionic acid, then reacted for 12 hours at 100 °C), and after that cooled down at normal temperature. Add 200g of purified water and ethylacetate to reaction solution respectively, concentrated after the extraction of organic layer. After crystallization by adding 250g of hexane, filtering the generated crystal, and drying up, 33.3g of 2-[(4-chloromethyl)phenyl]propionic acid was obtained.

Yield ratio on this experiment was 84%, and purity ratio measured by HPLC was 98.0%. Result of measurement by NMR(CDCl₃, ppm) was 1.5(3H, d), 3.7(1H, q), 4.7(2H, s), 7.3(4H, dd).

### EXAMPLE 8

162g of hydrochloric acid, 75g of potassium chloride, 3g of tetramethyl ammonium chloride, 18g of paraformaldehyde were poured orderly into methyl2-phenylpropionate, then reacted for 12 hours at 80 °C, and after that cooled down at normal temperature. Add 200g of purified water and ethylacetate to reaction solution respectively, concentrated after the extraction of organic layer, 29.5g of methyl[(4-chloromethyl)phenyl]propionate was obtained by distilling the reaction solution.

Yield ratio on this experiment was 74.3%, and purity ratio measured by HPLC was 98.5%. Result of measurement by NMR(CDCl₃, ppm) was 1.5(3H, d), 3.65(3H, s), 3.7(1H, q), 4.7(2H, s), 7.3(4H, dd).

## Claims

1. A process for the production of benzene derivatives expressed in below chemical formula (I) **characterized by** making reaction between a compound of chemical formula (II) and a hydroformylating agent in the presence of a halogenation agent and in the presence of a phase transfer catalyst; 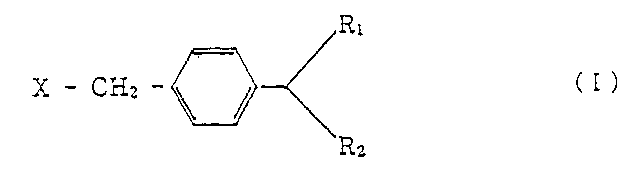 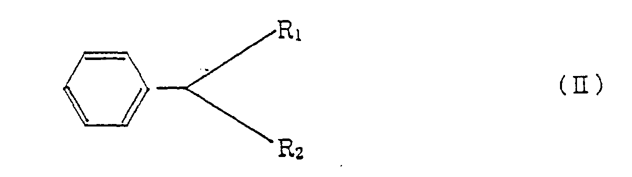 in chemical formulae (I) and (II), X is a halogen atom, R₁ is hydrogen atom or low alkyl radical of carbon number 1-6, R₂ is hydrogen atom or COOR₃, and R₃ is hydrogen atom or low alkyl radical of carbon number 1-6.

2. The process according to claim 1, wherein the hydroformylating agent is a polymer capable of generating formaldehyde in gaseous state or in solution state, paraformaldehyde, formaline, trioxane or methylal.

3. The process according to any of claim 1 or 2, wherein the halogenation agent is hydrochloric acid, hydrobromic acid, hydroiodic acid or metal halide in the presence of an acid.

4. The process according to any of claims 1-3, wherein the phase transfer catalyst is a tetraalkylammonium halide.

5. The process according to any of claims 1-4, wherein the quantity of the hydroformylating agent to be added is 1.0-10.0 equivalent per 1 equivalent of the compound of chemical formula (II).

6. The process according to any of claims 1-5, wherein the quantity of said halogenation agent to be added is 1.2-20 equivalent per 1 equivalent of the compound of chemical formula (II).

7. The process according to any of claims 1-6, wherein the reaction temperature is 40-140°C, and the reaction time is 2-30 hours.

8. The process according to any of claims 1-7, wherein the benzene derivative is 2-(4-chloromethylphenyl) propionic acid, 2-(4-chloromethylphenyl) propionic acid methyl ester, 2-(4-chloromethylphenyl) propionic ethyl ester, 2-(4-bromomethylphenyl) propionic acid, 2-(4-bromomethylphenyl) propionic methyl ester, or 2-(4-bromomethylphenyl) propionic ethyl ester.

## Patentansprüche

1. Verfahren zur Herstellung von Benzolderivaten der nachfolgenden chemischen Formel (I), **gekennzeichnet durch** die Umsetzung zwischen einer Verbindung der chemischen Formel (II) und einem Hydroformylierungsmittel in Gegenwart eines Halogenierungsmittels und in Gegenwart eines Phasentransferkatalysators; 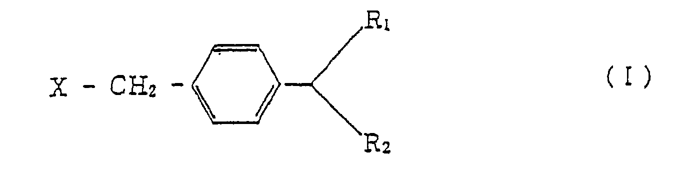 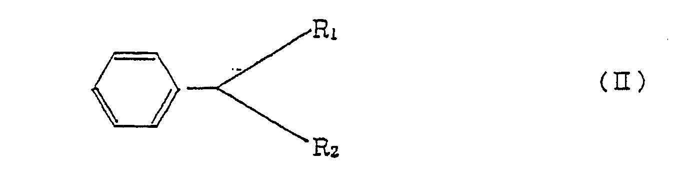 in den chemischen Formeln (I) und (II) sind X ein Halogenatom, R₁ Wasserstoffatom oder ein Niederalkylrest mit einer Kohlenstoffanzahl von 1-6, R₂ Wasserstoffatom oder COOR₃, und R₃ ist Wasserstoffatom oder Niederalkylrest mit einer Kohlenstoffanzahl von 1-6.

2. Verfahren nach Anspruch 1, wobei das Hydroformylierungsmittel ein Polymer, das fähig ist zur Erzeugung von Formaldehyd in gasförmigem Zustand oder im Lösungszustand, Paraformaldehyd, Formalin, Trioxan oder Methylal ist.

3. Verfahren nach Anspruch 1 und/oder 2, wobei das Halogenierungsmittel Chlorwasserstoffsäure, Bromwasserstoffsäure, Iodwasserstoffsäure oder ein Metallhalogenid in Gegenwart einer Säure ist.

4. Verfahren nach mindestens einem der Ansprüche 1-3, wobei der Phasentransferkatalysator ein Tetraalkylammoniumhalogenid ist.

5. Verfahren nach mindestens einem der Ansprüche 1-4, wobei die Menge des zuzusetzenden Hydroformylierungsmittels 1,0-10,0 Äquivalente pro 1 Äquivalent der Verbindung der chemischen Formel (II) beträgt.

6. Verfahren nach mindestens einem der Ansprüche 1-5, wobei die Menge des zuzusetzenden Halogenierungsmittels 1,2-20 Äquivalente pro 1 Äquivalent der Verbindung der chemischen Formel (II) beträgt.

7. Verfahren nach mindestens einem der Ansprüche 1-6, wobei die Umsetzungstemperatur 40-140°C und die Umsetzungszeit 2-30 Stunden betragen.

8. Verfahren nach mindestens einem der Ansprüche 1-7, wobei das Benzolderivat 2-(4-Chlormethylphenyl)-propionsäure, 2-(4-Chlormethylphenyl)-propionsäuremethylester, 2-(4-Chlormethylphenyl)-propionsäureethylester, 2-(4-Bromomethylphenyl)-propionsäure, 2-(4-Bromomethylphenyl)-propionsäuremethylester, oder 2-(4-Bromomethylphenyl)-propionsäureethylester ist.

## Revendications

1. Procédé pour la fabrication de dérivés de benzène représentés par la formule chimique (I) donnée ci-dessous, **caractérisé par** la mise en réaction d'un composé de formule chimique (II) et d'un agent d'hydroformylation en présence d'un agent d'halogénation et en présence d'un catalyseur de transfert de phase ; 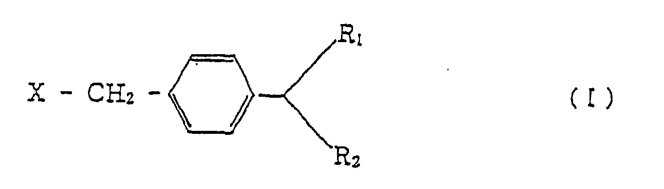 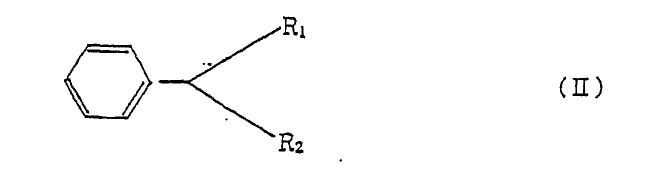 dans les formules chimiques (I) et (II), X est un atome d'halogène, R₁ est un atome d'hydrogène ou un radical alkyle inférieur avec un nombre de carbone de 1-6, R₂ est un atome d'hydrogène ou COOR₃, et R₃ est un atome d'hydrogène ou un radical alkyle inférieur avec un nombre de carbone de 1-6.

2. Procédé selon la revendication 1, **caractérisé en ce que** l'agent d'hydroformylation est un polymère capable de produire du formaldéhyde à l'état gazeux ou à l'état de solution, du paraformaldéhyde, du formol, du trioxane ou du méthylal.

3. Procédé selon l'une quelconque des revendications 1 à 2, **caractérisé en ce que** l'agent d'halogénation est l'acide chlorhydrique, l'acide bromhydrique, l'acide iodhydrique, ou un halogénure de métal en présence d'un acide.

4. Procédé selon l'une quelconque des revendications 1 à 3, **caractérisé en ce que** le catalyseur de transfert de phase est un halogénure d'ammonium tétraalkylé.

5. Procédé selon l'une quelconque des revendications 1 à 4, **caractérisé en ce que** la quantité d'agent d'hydroformylation à ajouter est de 1,0-10,0 équivalents pour 1 équivalent de composé de formule chimique (II).

6. Procédé selon l'une quelconque des revendications 1 à 5, **caractérisé en ce que** la quantité dudit agent d'halogénation à ajouter est de 1,2-20 équivalents pour 1 équivalent de composé de formule chimique (II).

7. Procédé selon l'une quelconque des revendications 1 à 6, **caractérisé en ce que** la température de réaction est de 40-140°C, et la durée de réaction de 2-30 heures.

8. Procédé selon l'une quelconque des revendications 1 à 7, **caractérisé en ce que** le dérivé de benzène est l'acide 2-(4-chlorométhylphényl) propionique, l'ester de méthyle d'acide 2-(4-chlorométhylphényl) propionique, l'ester d'éthyle d'acide 2-(4-chlorométhylphényl) propionique, l'acide 2-(4-bromométhylphényl) propionique, l'ester de méthyle 2-(4-bromométhylphényl) propionique ou l'ester d'éthyle 2-(4-bromométhylphényl) propionique.
